# EUROPEAN PATENT APPLICATION

(11) **EP 2 375 253 A1**
(43) Date of publication of application: **12.10.2011**
(21) Application number: 10729159.3
(22) Date of filing: 05.01.2010
(51) Int. Cl.: G01N 33/569, G01N 33/543

(54) **METHOD FOR ASSAYING ALL TYPES OF INFLUENZA VIRUSES**

(30) Priority: 07.01.2009 JP 2009001328
(71) Applicant: Otsuka Pharmaceutical Co., Ltd., Chiyoda-ku Tokyo 101-8535 (JP)
(72) Inventor: MASUDA, Hisayo, Osaka-shi Osaka 541-0045 (JP); ISHIKAWA, Yuichi, Osaka-shi Osaka 541-0045 (JP); SATO, Ayumi, A-1180 Wien 18 (AT); TANAKA, Hideaki, Osaka-shi Osaka 541-0045 (JP)
(74) Representative: Hartz, Nikolai
(86) International application number: PCT/JP2010/000023
(87) International publication number: WO 2010/079739

(57) **Abstract**

Provided is an immunoassay method whereby all *Haemophilus influenzae* strains can be simultaneously detected at a high sensitivity. Specifically provided is a method for immunoassaying all *Haemophilus influenzae* strains **characterized in that** an antibody that recognizes P4 antigen or P6 antigen of influenza viruses is used.

SEQUENCE LISTING

<110>  OTSUKA PHARMACEUTICAL CO., LTD.

<120>  Assay for Haemophilus influenzae

<130>  OP0058

<150>  JP2009-001328
<151>  2009-01-07

<160>  6

<170>  PatentIn version 3.1

<210>  1
<211>  867
<212>  DNA
<213>  Haemophilus influenzae

<220>
<221>  CDS
<222>  (68)..(529)
<223>

<400>  1
cccaagtaaa atttccagct tggtctccat acttaactaa ataaaaaact catttaggag      60

aaatcta atg aac aaa ttt gtt aaa tca tta tta gtt gca ggt tct gta      109
        Met Asn Lys Phe Val Lys Ser Leu Leu Val Ala Gly Ser Val
        1               5                   10

gct gca tta gcg gct tgt agt tcc tct aac aac gat gct gca ggc aat      157
Ala Ala Leu Ala Ala Cys Ser Ser Ser Asn Asn Asp Ala Ala Gly Asn
15                  20                  25                  30

ggt gct gct caa act ttt ggc gga tac tct gtt gct gat ctt caa caa      205
Gly Ala Ala Gln Thr Phe Gly Gly Tyr Ser Val Ala Asp Leu Gln Gln
                35                  40                  45

cgt tac aac acc gta tat ttt ggt ttt gat aaa tac gac atc acc ggt      253
Arg Tyr Asn Thr Val Tyr Phe Gly Phe Asp Lys Tyr Asp Ile Thr Gly
            50                  55                  60

gaa tac gtt caa atc tta gat gcg cac gca gca tat tta aat gca acg      301
Glu Tyr Val Gln Ile Leu Asp Ala His Ala Ala Tyr Leu Asn Ala Thr
        65                  70                  75

cca gct gct aaa gta tta gta gaa ggt aat act gat gaa cgt ggt aca      349
Pro Ala Ala Lys Val Leu Val Glu Gly Asn Thr Asp Glu Arg Gly Thr
    80                  85                  90

cca gaa tac aac atc gca tta gga caa cgt cgt gca gat gca gtt aaa      397
Pro Glu Tyr Asn Ile Ala Leu Gly Gln Arg Arg Ala Asp Ala Val Lys
95                  100                 105                 110

ggt tat tta gca ggt aaa ggt gtt gat gct ggt aaa tta ggc aca gta      445
Gly Tyr Leu Ala Gly Lys Gly Val Asp Ala Gly Lys Leu Gly Thr Val
                115                 120                 125

tct tac ggt gaa gaa aaa cct gca gta tta ggt cac gat gaa gct gca      493
Ser Tyr Gly Glu Glu Lys Pro Ala Val Leu Gly His Asp Glu Ala Ala
            130                 135                 140

tat tct aaa aac cgt cgt gca gtg tta gcg tac taa ttcttggtat           539
Tyr Ser Lys Asn Arg Arg Ala Val Leu Ala Tyr
        145                 150

ttctaatact tgaaaaacag gatccatttt ttattggatc ctgttttgtt ttcatcgttt    599

gtaatttaac caattagctt gaaagaatga atttattctt ttgattctaa aataaatgcg 659

ttatcattaa ctcatcaaca cagtgggtcg ttagctcagt cggtagagca gcggactttt 719

aatccgttgg tcgaaggttc gaatccttca cgacccacca ctctctgatt taattgtcca 779

gttgggttgt tagctcagtt ggtagagcag cggactctta attcgtcggt cgagagttcg 839

agcctctcac aacctaccat tcttaccg 867

<210> 2
<211> 1143
<212> DNA
<213> Haemophilus influenzae

<220>
<221> CDS
<222> (149)..(973)
<223>

<400> 2
gaattcttaa aaggaataaa ataatttcta ttataaccgt aggtagttta tttacggtta 60

ttaatgccat ctatgatgaa aaacactttt caattgaaaa gtgtttttca gaaagactta 120

ctataccctg aatgaatagg aacataat atg aaa aca acg tta aaa atg acc 172
Met Lys Thr Thr Leu Lys Met Thr
1               5

gca ctt gcg gct ctt tct gct ttt gtt tta gct ggc tgt ggt tca cac 220
Ala Leu Ala Ala Leu Ser Ala Phe Val Leu Ala Gly Cys Gly Ser His
    10                  15                  20

caa atg aaa tca gaa gaa cat gca aat atg caa tta caa caa caa gcg 268
Gln Met Lys Ser Glu Glu His Ala Asn Met Gln Leu Gln Gln Gln Ala
25                  30                  35                  40

gtg ctt gga tta aac tgg atg caa gat tct ggc gaa tat aaa gca tta 316
Val Leu Gly Leu Asn Trp Met Gln Asp Ser Gly Glu Tyr Lys Ala Leu
                45                  50                  55

gct tat caa gcg tac aat gcg gca aaa gtt gca ttt gat cac gca aaa 364
Ala Tyr Gln Ala Tyr Asn Ala Ala Lys Val Ala Phe Asp His Ala Lys
            60                  65                  70

gtg gca aaa ggt aag aaa aaa gcg gtt gtg gct gat tta gat gaa act 412
Val Ala Lys Gly Lys Lys Lys Ala Val Val Ala Asp Leu Asp Glu Thr
        75                  80                  85

atg tta gac aac agc cct tat gct ggc tgg caa gtt caa aat aac aaa 460
Met Leu Asp Asn Ser Pro Tyr Ala Gly Trp Gln Val Gln Asn Asn Lys
    90                  95                  100

cca ttc gat ggt aaa gat tgg act cgt tgg gta gac gca cgt caa tct 508
Pro Phe Asp Gly Lys Asp Trp Thr Arg Trp Val Asp Ala Arg Gln Ser
105                 110                 115                 120

cgt gcc gtt ccg ggt gcg gta gaa ttt aat aat tat gta aac agc cac 556
Arg Ala Val Pro Gly Ala Val Glu Phe Asn Asn Tyr Val Asn Ser His
                125                 130                 135

aac ggt aaa gtg ttc tac gta aca aac cgc aaa gac agc act gaa aaa 604
Asn Gly Lys Val Phe Tyr Val Thr Asn Arg Lys Asp Ser Thr Glu Lys
            140                 145                 150

tca ggc act atc gat gat atg aaa cgc tta ggt ttc aat ggc gtg gaa 652
Ser Gly Thr Ile Asp Asp Met Lys Arg Leu Gly Phe Asn Gly Val Glu
        155                 160                 165

gaa tct gca ttt tat ttg aaa aaa gac aaa tca gct aaa gcg gct cgt     700
Glu Ser Ala Phe Tyr Leu Lys Lys Asp Lys Ser Ala Lys Ala Ala Arg
    170                 175                 180

ttt gca gaa att gaa aaa caa ggc tat gaa atc gta ctt tat gta ggt     748
Phe Ala Glu Ile Glu Lys Gln Gly Tyr Glu Ile Val Leu Tyr Val Gly
185                 190                 195                 200

gat aac tta gat gac ttc ggt aat acc gta tat ggc aaa tta aac gct     796
Asp Asn Leu Asp Asp Phe Gly Asn Thr Val Tyr Gly Lys Leu Asn Ala
                205                 210                 215

gac cgc cgt gca ttc gtt gat caa aac caa ggc aaa ttt ggt aaa act     844
Asp Arg Arg Ala Phe Val Asp Gln Asn Gln Gly Lys Phe Gly Lys Thr
            220                 225                 230

ttc atc atg tta cct aac gca aac tac ggt ggc tgg gaa ggc ggt tta     892
Phe Ile Met Leu Pro Asn Ala Asn Tyr Gly Gly Trp Glu Gly Gly Leu
        235                 240                 245

gct gaa ggg tat ttc aaa aaa gat aca caa ggc caa atc aaa gct cgt     940
Ala Glu Gly Tyr Phe Lys Lys Asp Thr Gln Gly Gln Ile Lys Ala Arg
    250                 255                 260

tta gat gca gta caa gct tgg gat ggt aaa taa ttttccatta aaaagatcga     993
Leu Asp Ala Val Gln Ala Trp Asp Gly Lys
265                 270

tttaaatatc gattttgaaa atttaattga ggggctaagc tcagttttta ctggcttagc     1053

tttttcattt tcagtcactc aagtatcatc attactacat ctgagtttgc tatgatgttg     1113

cagcttcaaa tgatcaagta gagaatgacc                                      1143

<210>  3
<211>  33
<212>  DNA
<213>  Artificial

<220>
<223>  Designed DNA based on P6 gene of Haemophilus influenzae

<400>  3
gcgggatcct gtagttcctc taacaacgat gct                                  33

<210>  4
<211>  33
<212>  DNA
<213>  Artificial

<220>
<223>  Designed DNA based on P6 gene of Haemophilus influenzae

<400>  4
gcggagctcg tacgctaaca ctgcacgacg gtt                                  33

<210>  5
<211>  32
<212>  DNA
<213>  Artificial

<220>
<223>  Designed DNA based on P4 gene of Haemophilus influenzae

<400>  5
gcgggatcct gtggttcaca ccaaatgaaa tc                                   32

<210>  6
<211>  32
<212>  DNA
<213>  Artificial

<220>
<223>  Designed DNA based on P4 gene of Haemophilus influenzae

<400>  6
gcgctcgagt ttaccatccc aagcttgtac tg                                    32

## Description

### Technical Field

The subject invention relates to an assay method for specifically detecting all *Haemophilus influenzae* strains, and to an assay device employed therein.

### Background Art

*Haemophilus influenzae* is known as an indigenous bacterium in the human nasopharynx, similar to *Streptococcus pneumoniae*. Also, *Haemophilus influenzae* is known to cause, for example, respiratory tract infection, otitis media, meningitis, or sepsis, particularly in infants or children in which *Haemophilus influenzae* is not a resident bacterium. Similar to the other infectious bacteria, drug resistance of *Haemophilus influenzae* caused by frequent use of antibacterial drugs causes problems such as intractable or severe symptoms, and thus selection of an appropriate antibacterial drug at the start of therapy is increasingly recognized to be important. In principle, in infectious disease treatment, determination of a pathogenic bacterium as early as possible, and selection of an appropriate therapeutic drug are important for improvement of prognosis, reduction of medical cost, and prevention of appearance of drug-resistant bacteria. Therefore, provision of a diagnostic reagent for rapidly detecting *Haemophilus-influenzae*-derived common antigens is needed.

Commonly used techniques for detecting *Haemophilus influenzae* include Gram staining, microscopic examination, cultivation, color reaction by utilizing enzymatic metabolism of cells, latex agglutination, and PCR. A *Haemophilus influenzae* antigen detection kit (Slidex Meningite-Kit 5, product of bioMerieux), employing latex agglutination, has been provided as a rapid diagnostic technique. Although the kit detects polysaccharide antigens of *Haemophilus influenzae* type b, it cannot be used for detecting non-encapsulated *Haemophilus influenzae.* Meanwhile, a *Haemophilus influenzae* gene detection assay employing PCR, and such a gene detection assay which targets the P6 or P4 antigen of *Haemophilus influenzae* (i.e., a common antigen of *Haemophilus influenzae* strains) have been provided. However, the gene diagnostic assay employing PCR is currently less common because facilities where such an assay can be performed are limited. Also, a method employing a monoclonal antibody to the P6 antigen, in which *Haemophilus influenzae* is detected through western blotting (Non-Patent Document 1), has been reported. However, sensitivity of the method is so low that it cannot be used for clinical examination.

*Haemophilus influenzae* is a Gram-negative bacillus, and is divided into encapsulated strains and non-encapsulated strains according to presence or absence of a capsule covering the bacterium. The encapsulated strains are classified into six serotypes (types from a to f) according to the structural difference of polysaccharide antigens present in the capsule. The non-encapsulated strains are classified as nontypable. Polyribosylribitol phosphate, which is a type b capsular polysaccharide, has been reported as a type b-specific antigen, however, such a polysaccharide as a specific antigen has not been found in the non-encapsulated strains. P2 protein, which is an extracellular membrane protein, has been known as a major antigen of *Haemophilus influenzae* strains. However, the antigen is frequently mutated, so antigenicity differs between strains.

### Prior Art Document

### Non-Patent Document

Non-Patent Document 1: Journal of Clinical Microbiology (1989) 2263-2267

### Summary of the Invention

### Problems to be solved by the Invention

As described above, common *Haemophilus influenzae* antigens have not yet been fully found, and thus, any immunological methods capable of detecting all *Haemophilus influenzae* strains through a single procedure have not yet been provided.
An object of the subject invention for solving the aforementioned problems is to provide an immunological assay method capable of detecting all *Haemophilus influenzae* strains through a single procedure with high sensitivity. Another object of the subject invention is to provide an assay device employing the method.

### Means for Solving the Problems

In order to solve the aforementioned objects, the subject inventors have conducted extensive studies, and have focused on P4 and P6 proteins, both are known to be present in *Haemophilus influenzae* and to be relatively less likely to be mutated. However, the expression level of these proteins in *Haemophilus influenzae* is lower than the other antigens, therefore, even when antibodies recognizing the proteins are prepared, detection of all *Haemophilus influenzae* strains with high sensitivity by means of such antibodies has been considered to be difficult. Further, a lot of bacteria possess proteins exhibiting high degree of homology with P4 and P6 proteins, therefore, specific detection of *Haemophilus influenzae* has been considered to be difficult. However, beyond our expectations, the subject inventors have succeeded in preparation of an antibody which can highly selectively recognize P4 or P6 protein. Also, the subject inventors have found that such an antibody is less likely to be affected by, for example, contamination of bacteria such as *Pseudomonas aeruginosa* or *Escherichia coli*, which may be easily contaminated into the assay system during assay. The subject invention has been accomplished according to these findings.

Accordingly, the subject invention provides an immunological assay method for detection of all *Haemophilus influenzae* strains, characterized by comprising employing an antibody which recognizes P4 antigen or P6 antigen of *Haemophilus influenzae*.
The subject invention also provides an immunological assay device for detection of all *Haemophilus influenzae* strains, characterized by comprising an antibody which recognizes P4 antigen or P6 antigen of *Haemophilus influenzae*.

### Effects of the Invention

The immunological assay device can be provided through establishment of the high-sensitivity assay method of the subject invention, which is applicable to all *Haemophilus influenzae* strains. The assay device realizes convenient and rapid detection of all *Haemophilus influenzae* strains infected in the case of, for example, otitis media. The method of the subject invention is particularly useful for detection of otitis media caused by *Haemophilus influenzae*.

### Brief Description of the Drawings

[Fig. 1]

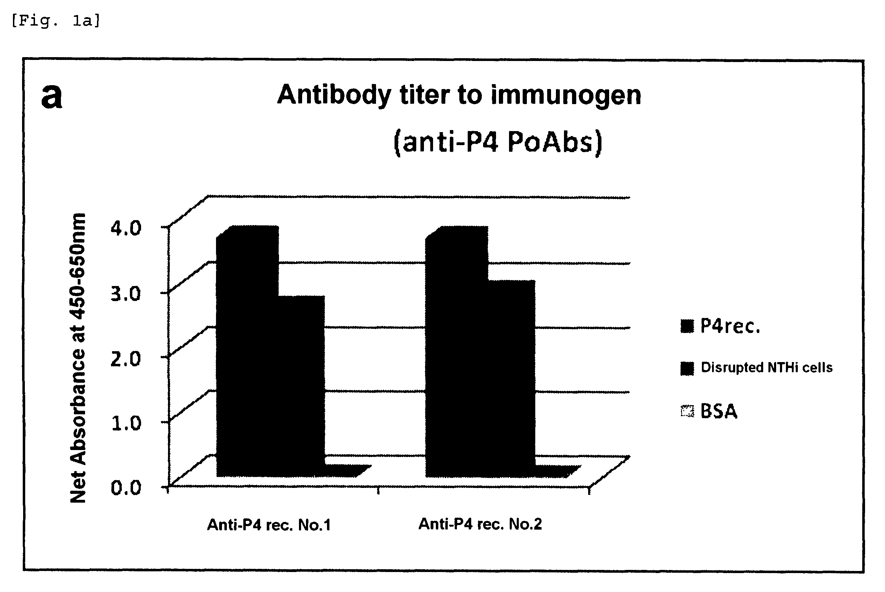

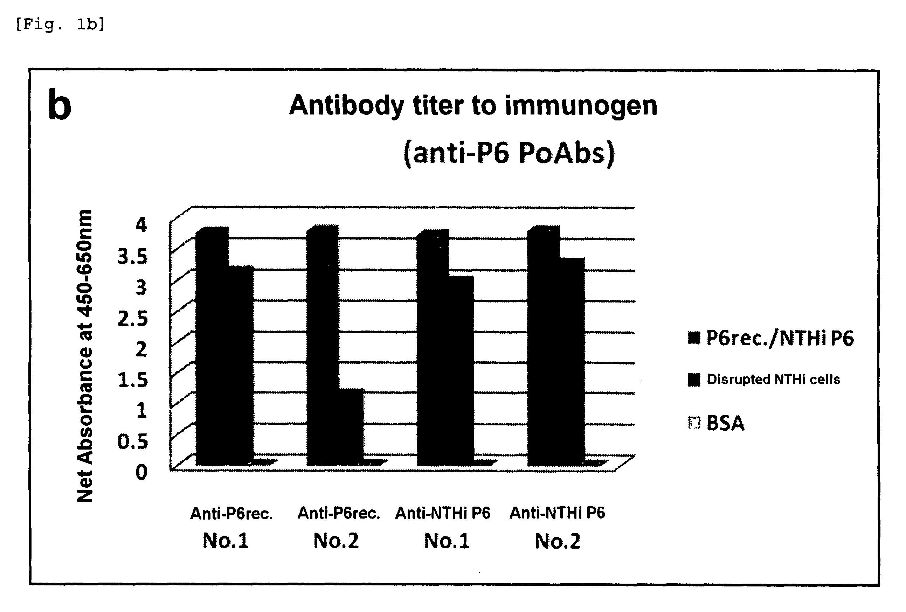

   Fig. 1 shows reactivity of anti-P4 PoAbs (1a) or anti-P6 PoAbs (1b) to an immunogen (i.e., a common antigen of *Haemophilus influenzae* (P6 or P4)) in ELISA.
[Fig. 2]

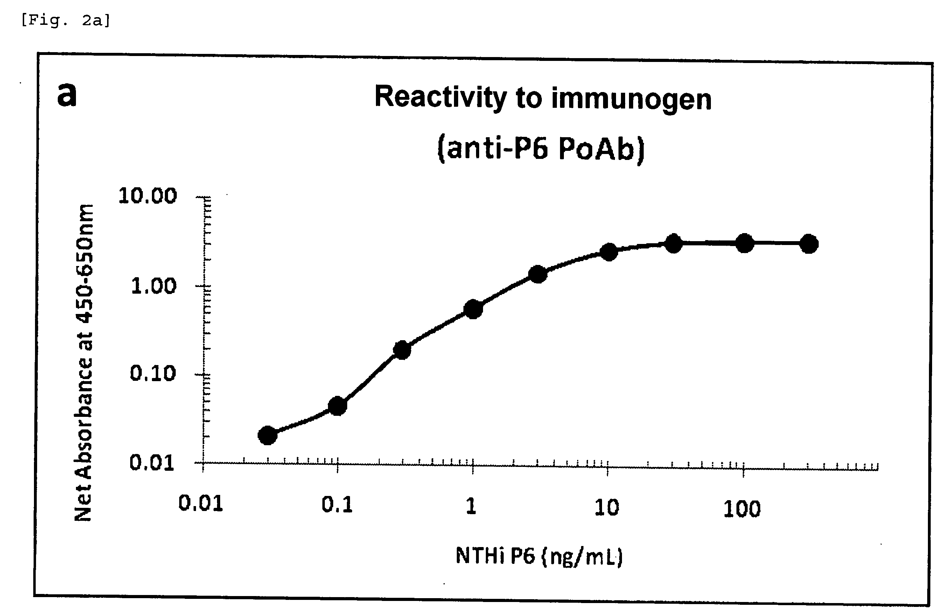

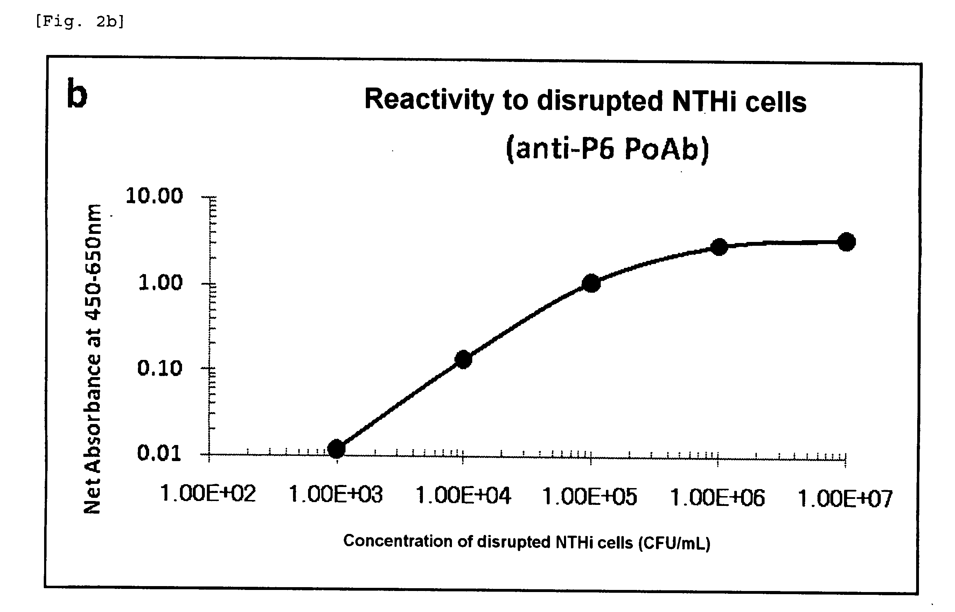

   Fig. 2 shows reactivity of an anti-P6 PoAb to NTHi P6 (2a) or disrupted cells of Non typable Haemophilus influenzae(NTHi) (2b) in ELISA.
[Fig. 3]

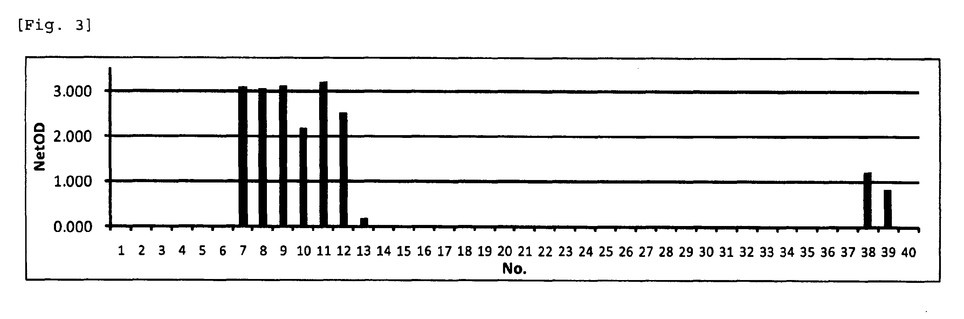

   Fig. 3 shows reactivity of an anti-P6 PoAb to various bacterial strains (Table 1) in ELISA.
[Fig. 4]

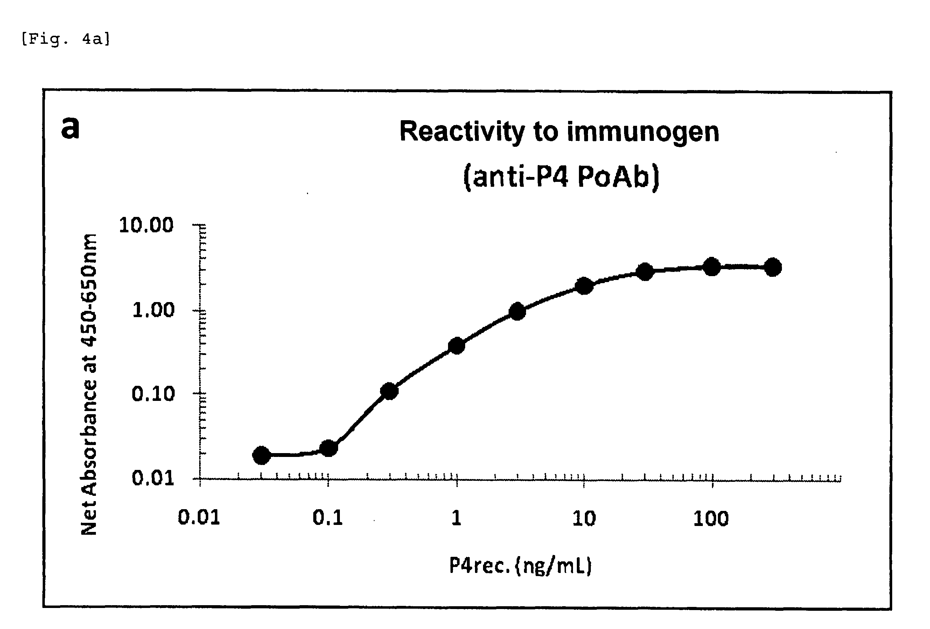

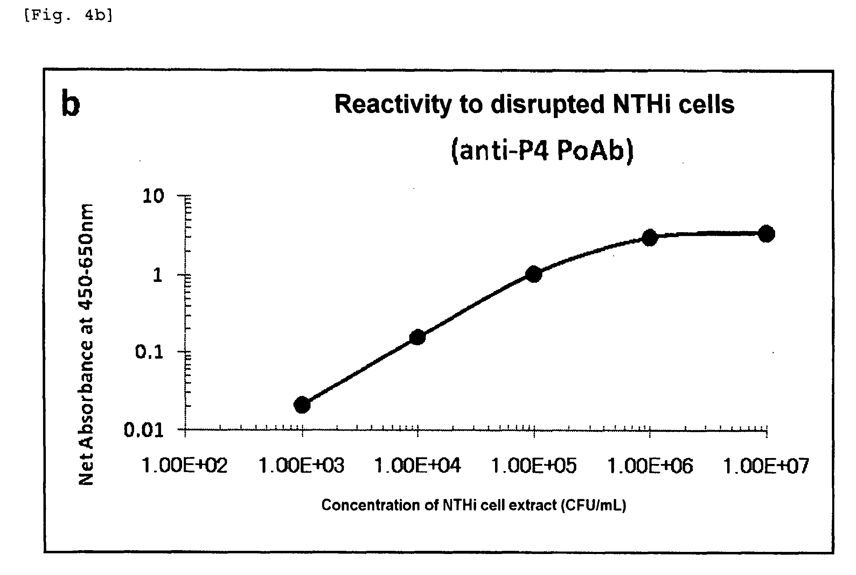

   Fig. 4 shows reactivity of an anti-P4 PoAb to recombinant P4 (4a) or disrupted cells of NTHi (4b) in ELISA.
[Fig. 5]

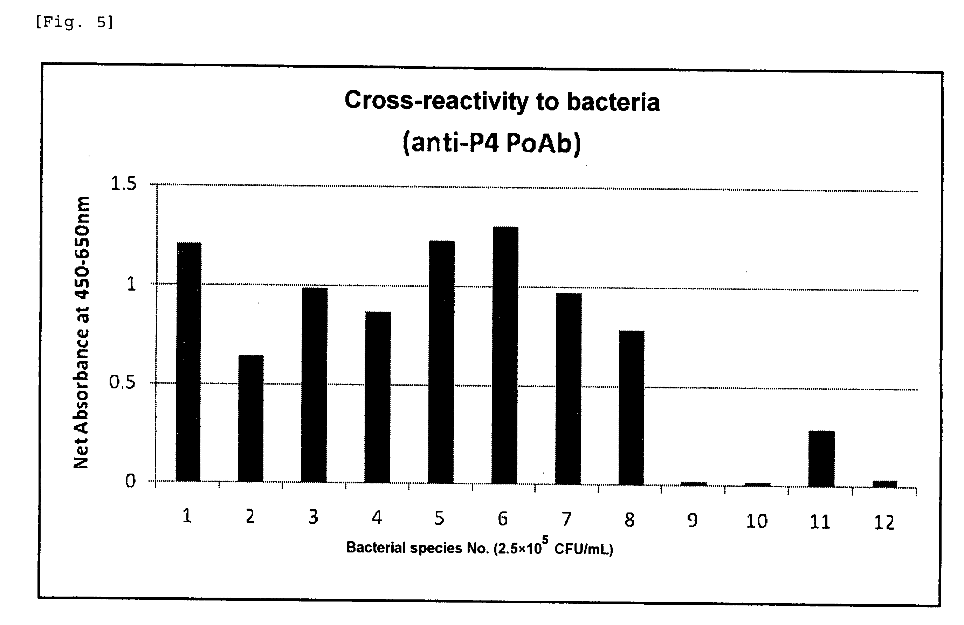

   Fig. 5 shows cross-reactivity of an anti-P4 PoAb to various bacterial strains (Table 1) in ELISA.
[Fig. 6]

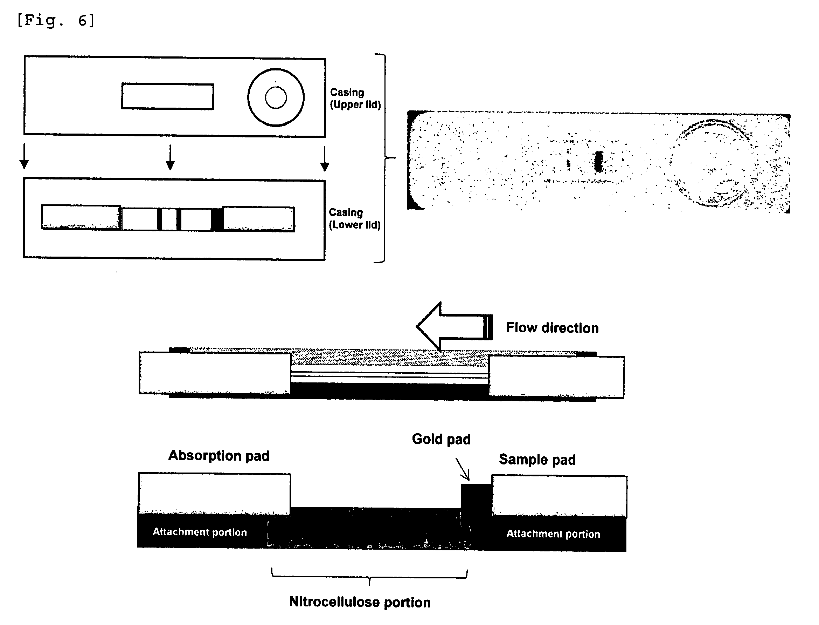

   Fig. 6 shows structure of a general immunochromatographic device.
[Fig. 7]

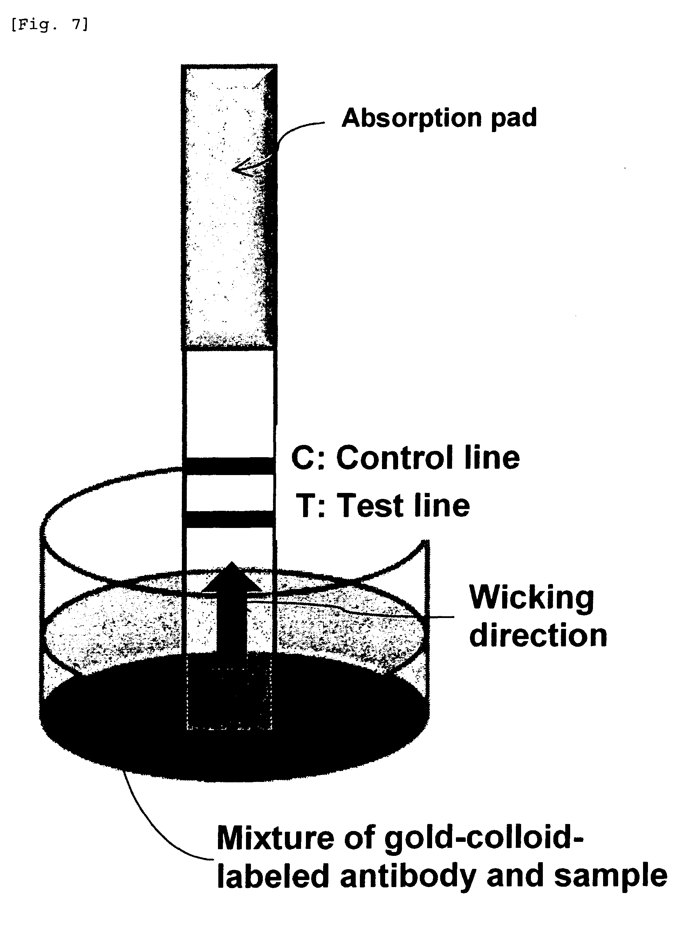

   Fig. 7 shows state where a sample is developed on an immunochromatographic strip.
[Fig. 8]

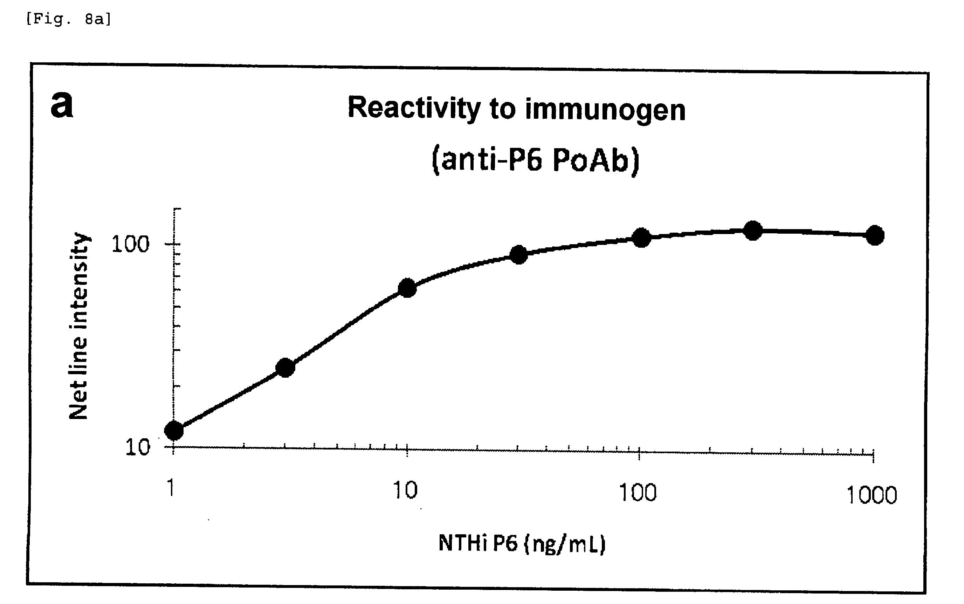

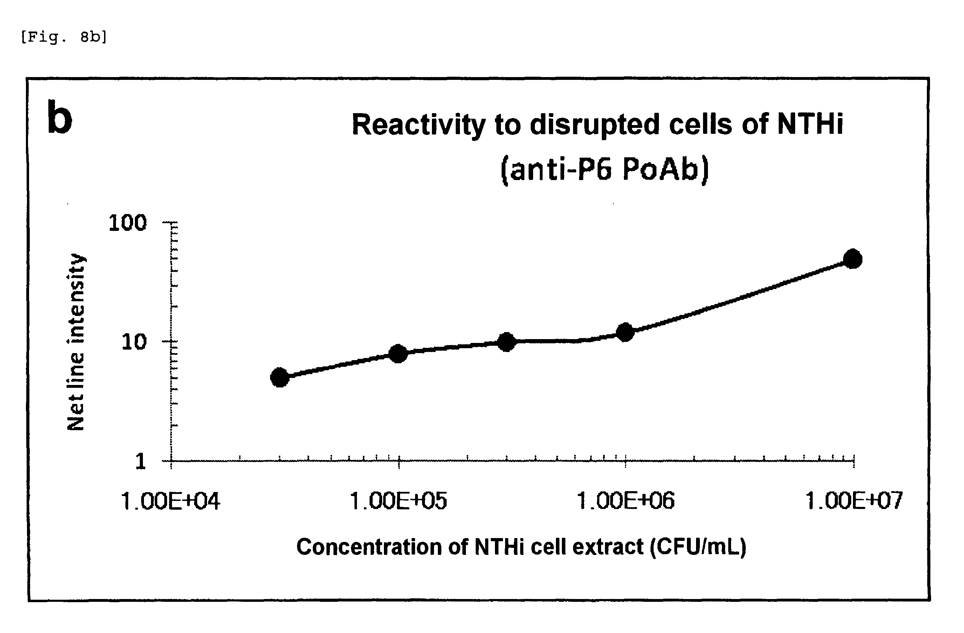

   Fig. 8 shows reactivity of an anti-P6 PoAb to NTHi P6 (8a) or disrupted cells of NTHi in immunochromatography (8b).

### Detailed Description of the Invention

The assay method of the subject invention is applied to *Haemophilus influenzae*, including encapsulated strains (from a to f types) and non-encapsulated strains. Specifically, the assay method is applied to their strains in which the P4 antigen or the P6 antigen is expressed. Diseases caused by *Haemophilus influenzae* include, for example, respiratory tract infection, otitis media, meningitis and sepsis in infants or children. In the case of such a disease, when a sample is collected from inflammatory area, the sample may be contaminated with other bacteria, and the bacteria may react with a component of the assay system. In contrast, a characteristic feature of the assay method of the subject invention is characterized in that the antibody employed does not react with bacteria containing, as a component, protein exhibiting homology with the P6 antigen or the P4 antigen; for example, *Haemophilus parainfluenzae*, *Pseudomonas aeruginosa*, or *Escherichia coli*. Generally, when a polyclonal antibody to a specific antigen is prepared and employed in an assay system, the antibody exhibits cross-reactivity with bacteria possessing an antigen exhibiting homology with the specific antigen. Therefore, an antibody to the P6 antigen or the P4 antigen is also expected to exhibit cross-reactivity with bacteria other than *Haemophilus influenzae*. Further, even when a polyclonal antibody to the P6 or P4 antigen (i.e., minor components of *Haemophilus influenzae*) can be prepared, the antibody is generally expected to be disadvantageous in terms of assay sensitivity. Therefore, there has not yet been reported an assay system employing a polyclonal antibody to the P6 antigen or the P4 antigen. However, in the case of the assay system of the subject invention employing such a polyclonal antibody, contrary to our expectations, the antibody exhibits significantly low cross-reactivity to bacteria which may be contaminated into a sample collected as described above; for example, *Haemophilus parainfluenzae*, *Pseudomonas aeruginosa*, or *Escherichia coli* (see Fig. 3). Thus, the method of the subject invention can detect all *Haemophilus influenzae* with higher sensitivity than expected.

The antibody employed in the subject invention, which recognizes the P4 antigen or the P6 antigen of *Haemophilus influenzae*, may be prepared by immunizing an animal with the P4 antigen or the P6 antigen, and collecting, for example, antiserum or eggs from the animal.

The P4 antigen or the P6 antigen employed for immunization may be obtained from *Haemophilus influenzae*, or may be prepared through recombination method. From the viewpoint of assay sensitivity, the P4 antigen or the P6 antigen extracted from *Haemophilus influenzae* is preferably employed. The P4 antigen or the P6 antigen may be whole P4 protein or P6 protein, or a partial polypeptide thereof.

Among the P4 antigen and the P6 antigen, the P6 antigen is preferably employed, from the viewpoints of specificity and sensitivity to *Haemophilus influenzae*.

The nucleotide sequence corresponding to the P6 antigen and the amino acid sequence encoded by the nucleotide sequence are shown in SEQ ID NO: 1 (accession No. M19391). The nucleotide sequence corresponding to the P4 antigen and the amino acid sequence encoded by the nucleotide sequence are shown in SEQ ID NO: 2 (accession No. M68502).

The followings are described for employing the P6 antigen, however, the P4 antigen can also be employed in a similar manner.

### Preparation of recombinant P6 or P4 antigen

Example of P6 antigen is hereinafter described. The P6 polynucleotide may be produced and obtained through chemical DNA synthesis on the basis of the sequence information of SEQ ID NO: 1, alternatively, may be readily produced and obtained through a common genetic engineering technique [see, for example, Molecular Cloning 2nd Ed., Cold Spring Harbor Lab. Press (1989); or Zoku Seikagaku Jikken Koza "Idenshi Kenkyuho I, II, III" edited by the Japanese Biochemical Society (1986)].
Examples of the chemical DNA synthesis include solid-phase synthesis employing phosphoramidite method. This synthesis method may employ an automated synthesizer.
In the case where a common genetic engineering technique is employed, specifically, the P6 polynucleotide may be produced by preparing a cDNA library according to usual method from an appropriate origin in which the P6 polynucleotide is expressed, and selecting a clone of interest from the cDNA library by using an appropriate probe or an antibody specific to the P6 polynucleotide [see, for example, Proc. Natl. Acad. Sci., USA., 78, 6613 (1981); or Science, 122, 778 (1983)].
The origin of cDNA is not particularly limited so long as the origin is a microorganism in which the P6 polynucleotide is expressed. Specifically, the origin employed is preferably *Haemophilus influenzae*.

Separation of total RNA from such an origin, separation and purification of mRNA, preparation and cloning of cDNA, or the other process may be carried out according to usual method. The method for screening the P6 polynucleotide from the cDNA library is not particularly limited, and this screening may be carried out according to usual method. Specific examples of the screening method include a method of selecting a corresponding cDNA clone through immunological screening employing an antibody specific to a polypeptide produced by the cDNA; a plaque hybridization method employing a probe which selectively binds to a nucleotide sequence of interest; a colony hybridization method; and combinations thereof.

The probe employed in aforementioned hybridization methods is generally, for example, a DNA fragment chemically synthesized on the basis of the nucleotide sequence information of the P6 polynucleotide. The probe employed for the aforementioned screening may be a sense primer and/or antisense primer designed on the basis of the nucleotide sequence information of the polynucleotide of the subject invention.

For preparation of the P6 polynucleotide, the DNA or RNA amplification method through PCR [Science, 130, 1350 (1985)] or a modification thereof may be suitably employed. Particularly when full-length cDNA is difficult to be obtained from the library, for example, the RACE method [Rapid amplification of cDNA ends; Jikken Igaku, 12 (6), 35 (1994)], in particular, the 5'-RACE method [M. A. Frohman, et al., Proc. Natl. Acad. Sci., USA., 8, 8998 (1988)] is suitably employed.

Primer employed for the aforementioned PCR may be appropriately designed on the basis of the sequence information of the P6 polynucleotide, and may be synthesized according to usual method. Isolation/purification of the thus-amplified DNA or RNA fragment may be carried out according to usual method as described above; for example, gel electrophoresis or hybridization method.

When the P6 polynucleotide is employed, an expression product of the polynucleotide (i.e., the aforementioned polypeptide) can be readily and reliably produced in a large amount according to usual genetic engineering technique.

### Expression vector

The expression vector employed is not particularly limited, so long as it contains the P6 polynucleotide and expresses the P6 polynucleotide. Generally, the expression vector is appropriately selected in consideration of the relationship between the vector and a host cell.

When the host cell employed is a prokaryotic cell, the expression vector may be, for example, an expression plasmid which can be replicated in the host cell and contains a promoter and an SD (Shine-Dalgarno) nucleotide sequence provided upstream of the aforementioned polynucleotide so that the polynucleotide can be expressed therein. Specific examples of the expression vector include expression plasmids having a P_{L} promoter, a T7 promoter, and a lac promoter. Examples of other preferred bacterial expression vectors include plasmids pKK233-2 and pKK233-3 having a tac promoter or a trc promoter. The expression vector employed is not limited to these examples, and any known strains or vectors may be used.

When the host cell employed is a vertebrate cell, the expression vector may be generally a vector containing, for example, a promoter located upstream of the aforementioned polynucleotide to be expressed, an RNA splice site, a polyadenylation site, or a transcription termination sequence. If necessary, such a vector may further contain a replication origin. Eukaryotic vectors useful for insertion of the aforementioned polynucleotide are commonly known. Examples of appropriate eukaryotic vectors include pCD and pCMV. Examples of other optional vectors include pMSG and pSVL employing an MMTV or SV40 late promoter.

### Recombinant cell

A recombinant cell (transformant) is produced through transformation by use of an expression vector containing the P6 polynucleotide.
The host cell employed for production of a recombinant cell may be a prokaryotic cell or a eukaryotic cell.
The prokaryotic cell employed as a host cell may be a bacterial cell widely used for gene recombination. Examples of the bacteria include *Escherichia coli*, *Streptomyces*, *Bacillus subtilis*, *Streptococcus*, and *Staphylococcus*. The bacteria are particularly preferably, for example, *Escherichia coli* or *Bacillus subtilis*.
Examples of the eukaryotic cell employed as a host cell include cells of eukaryotic microorganisms such as yeast and *Aspergillus*; insect cells such as *Drosophila* S2 cell and *Spodoptera* Sf9 cell; and animal and plant cells such as L cell, CHO cell, COS cell, HeLa cell, C127 cell, BALB/c3T3 cell (including mutant strains lacking, for example, dihydrofolate reductase or thymidine kinase), BHK21 cell, HEK293 cell, Bowes melanoma cell, and oocyte.

The method for incorporating the aforementioned expression vector into a host cell is not particularly limited, and any common method may be employed. For example, incorporation of the aforementioned expression vector into a host cell may be carried out through any technique described in many standard laboratory manuals, including Davis, et al. (BASIC METHODS IN MOLECULAR BIOLOGY, 1986) and Sambrook, et al. (MOLECULAR CLONING: A LABORATORY MANUAL, 2nd Ed., Cold Spring Harbor Laboratory Press, Cold Spring Harbor, N.Y., 1989). Specific examples of the technique include calcium phosphate transfection, DEAE-dextran-mediated transfection, transvection, microinjection, cationic-lipid-mediated transfection, electroporation, transduction, scrape loading, ballistic introduction, and infection.

### Production of P6 antigen peptide by use of recombinant cell

For production of the P6 polypeptide, P6-polynucleotide-incorporated recombinant cells may be cultured, and the P6 polypeptide may be collected from the thus-cultured cells and/or the resultant culture product.

Passage culture or batch culture may be carried out by use of a culture medium suitable for the host employed. Culturing may be carried out until an appropriate amount of the P6 polypeptide is produced inside and outside of the recombinant cells.
The culture medium employed for culturing may be appropriately selected from conventionally used ones in consideration of the host cell employed. Culturing may be carried out under conditions suitable for growth of the host cell.

For separation or purification of the thus-produced P6 polypeptide, the polypeptide may optionally be subjected to any separation method utilizing, for example, its physical or chemical properties [see, for example, "Biochemical Data Book II," pp. 1175-1259, 1st Edition, 1st Printing, June 23, 1980, published by Tokyo Kagaku Dojin; Biochemistry, 25 (25), 8274 (1986); and Eur. J. Biochem., 163, 313 (1987)]. Specific examples of the method include those similar to the method for isolation/purification of a protein of interest described below in the section "extraction and isolation of antigen."

### Method for extraction and isolation of P6 antigen peptide

Hereinafter, a method for isolation and purification of the P6 peptide from a microorganism having P6 polypeptide production ability is described. Firstly, microorganism cells having P6 polypeptide production ability are disrupted in order to prepare a crude extract from the microorganism. Cell disruption is carried out through commonly used cell disruption treatment method, such as treatment with a crusher (e.g., French press or cell mill) or ultrasonication in a hypotonic solution. The resultant crude extract may be supplemented with an appropriate buffer.

For more purification of the resultant crude extract, the crude extract may be further subjected to purification treatment, such as ammonium sulfate precipitation, organic solvent precipitation with, for example, ethanol, or isoelectric precipitation. Subsequently, the crude extract may be subjected to ion-exchange chromatography, gel filtration chromatography, hydrophobic chromatography, any affinity chromatography, reversed-phase chromatography, hydroxyapatite column chromatography etc., to thereby prepare a fraction containing the P6 polypeptide. Such chromatographic treatment may optionally employ an open column or HPLC. The purity of the thus-prepared fraction containing the P6 polypeptide may be conveniently estimated through electrophoresis (in particular, SDS-PAGE) in a visible manner. The P6 polypeptide may be determined through, for example, amino acid sequence analysis, mass spectrometry employing a mass spectrometer (e.g., MALDI-TOF MS, ESI Q-TOF MS, or MALDI Q-TOF MS), or peptide mass finger printing.

### Production of antibody

The P6 antigen peptide or P6-antigen-peptide-recognizing antibody employed for the assay may be produced through a customary method. Preferably, a polyclonal antibody is employed.
A polyclonal antibody may be produced through the following procedure: a warm-blooded animal (e.g., rabbit, sheep, guinea pig, or chicken) is immunized a plurality of times with an emulsion prepared by generally mixing the aforementioned antigen of interest with complete Freund's adjuvant, and the resultant antiserum is obtained through a commonly used method. In the case where a chicken is employed, the chicken is immunized a plurality of times with the aforementioned immunogen, and IgY is produced in an egg from the chicken. Thus-produced IgY is obtained from the yolk of the egg through a commonly used method.

### Construction of assay system

The *Haemophilus influenzae* assay method of the subject invention preferably employs a polyclonal antibody which recognizes the P6 antigen or the P4 antigen. The test sample employed may be *Haemophilus influenzae* or an extract of *Haemophilus influenzae*. Particularly preferably, a polyclonal antibody which recognizes the P6 antigen is employed.
Typical examples of the assay device employing the immunological method of the subject invention include ELISA and immunochromatography assay devices. Other preferred examples include a technique employing an antibody to the subject, such as radioimmunoassay (RIA).

### ELISA method

When, for example, the sandwich ELISA method is employed, generally, a primary antibody (anti-P6 polyclonal antibody or anti-P4 polyclonal antibody) is immobilized onto a 96-well plate, and a biological sample (blood, serum, plasma, or another body fluid component (in particular, middle ear fluid (e.g., discharge from the ear), pharyngeal swab, or urine)), or, if necessary, a preparation obtained by diluting such a sample with an appropriate buffer is added to the plate for contact with the antibody for a certain period of time, to thereby bind the antigen to the corresponding antibody. Thereafter, the plate is washed with an appropriate buffer, and then a labeled secondary antibody (labeled anti-P6 antibody or labeled anti-P4 antibody) is reacted with the antigen-antibody complex. When, for example, the labeling substance is biotin, peroxidase-labeled avidin (or streptavidin) is reacted with the labeled antibody, and an appropriate reaction substrate (e.g., TBM) is added for color development. After a certain period of time, colorimetric determination is carried out at a specific wavelength (in this case, 450 nm). The avidin-labeled antibody may be labeled with, for example, peroxidase (HRP), alkaline phosphatase, acid phosphatase, glucose oxidase or tyrosinase. The substrate employed is not particularly limited, so long as it is commercially available and is generally used.
The labeled antibody includes a biotin-labeled antibody (avidin or sterptavidin is used thereto), or the secondary antibody may be labeled with, for example, peroxidase (HRP), alkaline phosphatase, acid phosphatase, glucose oxidase, or tyrosinase. The substrate employed is not particularly limited, so long as it is commercially available and is generally used.

### Immunochromatography assay device

Immunochromatography may be carried out through a general technique shown in Fig. 6. A simple immunochromatographic device includes a sample applying portion (sample pad) attached at one end of a plastic sheet; a portion on which a gold-colloid-labeled anti-P6 PoAb is dry-fixed (gold pad); a nitrocellulose portion; and a portion which absorbs excess sample (absorption pad). The sample pad or the absorption pad is preferably formed of glass fiber filter paper, cellulose, cotton, or filter paper made of a mixture thereof. The nitrocellulose portion preferably has a pore size of 1.0 to 20 µm (more preferably 5.0 to 10.0 µm). When the gold-colloid-labeled anti-P6 PoAb is employed in the form of solution, the gold pad is not required.

On the test line of the nitrocellulose portion is applied the aforementioned P6 PoAb at a concentration of 0.1 to 10 mg/mL (preferably 0.2 to 5 mg/mL). On the control line, for example, a goat or mouse IgG having anti-rabbit IgG activity is applied at a concentration of 0.1 to 10 mg/mL. After drying, for example, a protein or a polymer is used for blocking. Proteins such as BSA, casein or gelatin, or a polymer such as polyvinyl alcohol (PVA), polyvinylpyrrolidone (PVP), or polyethylene glycol (PEG) may be used for blocking. Particle size of the gold colloid using the label of antibody is preferably 20 to 150 nm (more preferably 30 to 100 nm). The antibody may be labeled with the colloid through adsorption or covalent binding via, for example, another protein. The gold colloid may be replaced with colored latex particles or another noble metal colloid. Similar to the case of the nitrocellulose portion, blocking of the gold colloid labeled antibody may be carried out by use of a protein such as BSA, casein, or gelatin, or a polymer such as PVA, PVP, or PEG. Thus-assembled test strip may be placed in a plastic casing and employed for the assay.

### Diagnosis kit

The presence or absence of the infection of *Haemophilus influenzae* in, for example, an otitis media patient can be determined by measuring the amount of the P4 antigen or the P6 antigen contained in a test sample from the patient. According to the subject invention, a kit composition containing a reagent for immunochromatography or an ELISA kit can be provided. Particularly, the kit includes therein an antibody to the P4 antigen or the P6 antigen. The kit further includes, for example, albumin such as BSA, a secondary antibody, and an enzyme substrate (in the case where an enzyme is employed for labeling). When, for example, the labeled antibody is a biotin-labeled antibody, the assay kit may include peroxidase-labeled avidin as a secondary antibody. A kit for diagnosis of all *Haemophilus influenzae* strains including, for example, a substrate for the peroxidase may also be provided.

The method of the subject invention can specifically detect *Haemophilus influenzae* in various infectious diseases. Therefore, when an infected subject is diagnosed as positive by the method of the subject invention, the infection can be determined to be caused by *Haemophilus influenzae.* Examples of the disease to which the subject invention is applied include respiratory tract infection, otitis media, and sepsis in infants or children. Most cases of otitis media are not caused by infection with *Haemophilus parainfluenzae* (International Journal of Pediatric Otorhinolaryngology (2003) 67, 43-51). Therefore, the method of the subject invention is particularly useful for detecting *Haemophilus influenzae* strains in otitis media.

### Examples

The examples of the subject invention are described hereinafter, which should not be construed as limiting the invention thereto.

### 1. Production of anti-P6 or anti-P4 antibody

P6 protein or P4 protein was prepared through direct extraction from cells or prepared as a recombinant protein using *Escherichia col*i, and a rabbit was immunized with the prepared protein, to thereby produce a polyclonal antibody. Subsequently, an ELISA system was constructed by use of thus-produced antibody, and evaluated. In addition, an immunochromatography system employing the antibody was constructed, and determined whether or not the system can be used for rapid diagnosis.

### 1-1. Preparation of antigen

### 1-1-1. Preparation of P6 antigen

The P6 antigen was obtained through the following two processes: direct extraction from *Haemophilus influenzae* cells, and preparation by recombinant technology using *Escherichia coli*.

### Extraction from cells

The P6 antigen was extracted from cells according to the method of Kodama, et al. (Infection and Immunity, 68, 2294-2300 (2000)). Specifically, non-encapsulated *Haemophilus influenzae* cells (NTHi (ATCC No. 9333)) were cultured in a chocolate agar medium supplemented with bacitracin (product of BD), and thus-cultured cells were collected as precipitate through scraping and centrifugation. The precipitate was suspended in a buffer containing 1% SDS/0.1M Tris/0.5M NaCl/0.1% 2-mercaptoethanol (pH: 8.0), followed by sonication and then heating at 37°C for 30 minutes. The precipitate obtained through centrifugation was suspended in the aforementioned buffer supplemented with 10µg/mL RNase A (product of SIGMA), followed by sonication and heating at 37°C for 30 minutes. Subsequently, centrifugation was carried out, and the resultant precipitate was suspended in a buffer containing no RNase A, followed by sonication and heating. This procedure was carried out twice. Thereafter, centrifugation was carried out, and the resultant precipitate was suspended in a buffer containing 0.01M Tris/0.15M NaCl (pH: 7.4), followed by sonication and heating at 65°C for 30 minutes. The supernatant obtained through centrifugation was concentrated by means of centrifugal ultrafiltration, and the concentrate was employed as an antigen.

### Preparation by recombinant technology using Escherichia coli

The P6 gene (mature form) lacking a signal sequence of *Haemophilus influenzae* type b strain (Hib) (ATCC No. 10211) was amplified through direct PCR using 5'-GCGGGATCCTGTAGTTCCTCTAACAACGATGCT-3' (SEQ ID NO: 3) as a forward primer, and 5'-GCGGAGCTCGTACGCTAACACTGCACGACGGTT-3' (SEQ ID NO: 4) as a reverse primer. The PCR was carried out by means of GeneAmp^{™} PCR System 9700 (product of PE Applied Biosystems) employing TaKaRa rTaq (product of Takara). Thus-amplified fragment was inserted into a subcloning vector pCR^{™} 2.1 (product of Invitrogen). The sequence of the amplified P6 gene was confirmed, and subsequently a fragment cleaved at BamHI and XhoI sites was inserted into an expression vector pET21a (product of MERCK), to thereby prepare a plasmid for recombinant P6 expression (P6-pET21a). The plasmid was transfected into *Escherichia coli* BL21, and then expression of the recombinant P6 antigen was induced under the 1mM IPTG at 37°C condition for three hours. After precipitation of cells by centrifugation, the cells were lysed with BugBuster^{™} Protein Extraction Reagent (product of MERCK), and a soluble fraction was recovered through centrifugation. The histidine-tagged recombinant P6 antigen was applied to a Ni column (HisTrap^{™} HP, product of GE Healthcare) equilibrated with an equilibration buffer (100mM phosphate, 300mM NaCl, 50mM imidazole, pH: 7.8), and the column was washed sequentially with the equilibration buffer and a washing buffer (100mM phosphate, 300mM NaCl, 50mM imidazole, pH: 6), followed by elution with an elution buffer (100mM phosphate, 300mM NaCl, 200mM imidazole, pH 6). Thus-purified recombinant P6 antigen was dialyzed against D-PBS(-) at 4°C overnight and then concentrated through centrifugal ultrafiltration.

### 1-1-2. Preparation of P4 antigen

The P4 antigen was prepared only as *Escherichia coli* recombinant protein. Preparation was carried out following almost the same manner as in the case of the recombinant P6 antigen. Firstly, the P4 gene (mature form) lacking a signal sequence was amplified through direct PCR by using, as a template, cells of a non-encapsulated *Haemophilus influenzae* strain (NTHi) (ATCC No. 9333). The PCR was carried out by means of GeneAmp^{™} PCR System 9700 employing TaKaRa ExTaq (product of Takara), 5'-GCGGGATCCTGTGGTTCACACCAAATGAAATC-3' (SEQ ID NO: 5) as a forward primer, and 5'-GCGCTCGAGTTTACCATCCCAAGCTTGTACTG-3' (SEQ ID NO: 6) as a reverse primer. The thus-amplified fragment was treated with restriction enzymes BamHI and XhoI, and the cleaved fragment was inserted into BamHI and XhoI sites of an expression vector pET21a, to thereby prepare a plasmid for recombinant P4 expression (P4m-pET21a). The plasmid was transfected into *Escherichia coli* BL21, and then expression of the recombinant P4 antigen was induced under the 1mM IPTG, 37°C condition for three hours. After collection of cells by centrifugation, the cells were lysed with BugBuster^{™} Protein Extraction Reagent, and a soluble fraction was recovered through centrifugation. The histidine-tagged recombinant P4 antigen was applied to a Ni column equilibrated with the equilibration buffer, and the column was washed sequentially with the equilibration buffer and the washing buffer, followed by elution with the elution buffer. The thus-purified recombinant P4 antigen was dialyzed against D-PBS(-) at 4°C overnight and then concentrated through centrifugal ultrafiltration.

### 1-2. Production of antibody

Rabbits were subcutaneously immunized with each of the thus-prepared immunogens together with an adjuvant (e.g., Freund's adjuvant). Immunization (dose: 100 µg/body) was carried out five to six times every other week. Two individuals were immunized with each antigen, and whole blood collected from each individual was subjected to centrifugation. Thereafter, the resultant antiserum was frozen and stored. An appropriate amount of the antiserum was thawed and then purified using affinity purification using Protein A and gel filtration. The resultant antibody was employed as a polyclonal antibody (PoAb).

### 1-3. Reactivity of PoAb

The titer of each PoAb produced through the aforementioned method was evaluated through the below-described ELISA. Firstly, the recombinant P4 antigen, the recombinant P6 antigen, or the extracted P6 antigen, serving as an immunogen, was immobilized onto an ELISA plate (1 µg/mL), followed by blocking with, for example, bovine serum albumin (BSA). The anti-P4 PoAbs purified from two individuals were added (1 µg/mL each) to the recombinant-P4-antigen-immobilized plate for antigen-antibody reaction. The four anti-P6 PoAbs obtained by use of the two P6 antigens were added (1 µg/mL each) to the recombinant-P6-antigen-immobilized plate and the extracted-P6-antigen-immobilized plate for antigen-antibody reaction. After washing, each of the resultant antigen-antibody complexes was reacted with an anti-rabbit IgG antibody (product of ZYMED) labeled with horseradish peroxidase (HRP), followed by color development by use of 3,3',5,5'-tetramethylbenzidine (TMB). A BSA-immobilized plate was employed as a reference. For determination of the reactivity of each of the above-obtained PoAbs to cells, disrupted NTHi cells (ATCC No. 9333) obtained through, for example, surfactant treatment or sonication were immobilized onto a plate (1 µg/mL), and ELISA was carried out in a manner similar to that described above. The results are shown in Figs. 1a and 1b. Both anti-P4 and anti-P6 PoAbs were found to show reactivity not only to the corresponding immunogens, but also to the disrupted cells, which suggested that these PoAbs are possible to be used for construction of a sandwich ELISA system for detecting *Haemophilus influenzae* antigens.

### 2. Construction of ELISA system for detection of P6 or P4 antigen, and evaluation of their performance (ELISA example) 2-1. Sandwich ELISA for detection of P6 antigen

The anti-P6 PoAb (anti-NTHi P6 PoAb No. 2) was immobilized onto an ELISA plate (5 µg/mL), followed by blocking with, for example, BSA. The immunogen or disrupted cells which had been appropriately diluted stepwise were added to the antibody-immobilized plate for reaction. After washing, the antigen-antibody complex was reacted with a biotin-labeled antibody prepared through labeling of the same type of PoAb as the immobilized anti-P6 PoAb. After washing again, HRP-labeled streptavidin was added to the plate for reaction, followed by color development with TMB. Figs. 2a and 2b show examples of dose-dependent curves. As shown therein, the P6 antigen was detected at 30 pg/mL or more, and disrupted NTHi cells (ATCC No. 8149) were detected at 10³ to 10⁷ CFU/mL.

### 2-2. Cross-reactivity of sandwich ELISA for detection of P6 antigen

The cross-reactivity of the anti-P6 PoAb to a variety of bacteria shown in Table 1 was evaluated through the aforementioned sandwich ELISA. The results are shown in Fig. 3. All *Haemophilus influenzae* strains (including both encapsulated and non-encapsulated strains) were successfully detected.

**[Table 1]**

| No. | ATCC No. | | No. | ATCC No. | |
|---|---|---|---|---|---|
| 1 | 25285 | *Bacteroides fragilis* | 21 | 15032 | *Prebotella intermedia* |
| 2 | BAA-589 | *Bordetella pertussis* | 22 | 25845 | *Prebotella melaninogenica* |
| 3 | 66396 | *Candida albicans* | 23 | 9027 | *Pseudomonas aeruginosa* |
| 4 | 10700 | *Corynebacterium pseudodiphtheriticum* | 24 | 700699 | *Staphylococcus aureus* |
| 5 | 14116 | *Cryptococcus neoformants* | 25 | 14776 | *Staphylococcus aureus* |
| 6 | 8486 | *Eubacterium limosum* | 26 | 33397 | *Streptococcus anginosus* (group G) |
| 7 | 9006 | *Haemophilus influenzae,a* | 27 | 12386 | *Streptococcus agalactiae* (group B) |
| 8 | 10211 | *Haemophilus ingluenzae,b* | 28 | 27513 | *Streptococcus constellaus* |
| 9 | 9007 | *Haemophilus influenzae,c* | 29 | 27823 | *Streptococcus constellaus* |
| 10 | 9008 | *Haemophilus influenzae,d* | 30 | 9528 | *Streptococcus equi* (group C) |
| 11 | 8142 | *Haemophilus influenzae,e* | 31 | 9895 | *Streptococcus intermedius* |
| 12 | 700222 | *Haemophilus influenzae,f* | 32 | 27335 | *Streptococcus intermedius* |
| 13 | 7901 | *Haemophilus parainfluenzae* | 33 | 49456 | *Streptococcus mitis* |
| 14 | 9997 | *Klebsilla pneumoniae* | 34 | 35037 | *Streptococcus oralis* (group A) |
| 15 | 33152 | *Legionella pneumophila* | 35 | 6303 | *Streptococcus pneumoniae* |
| 16 | 33153 | *Legionella pneumophila* | 36 | 10556 | *Streptococcus sanguis* |
| 17 | 33216 | *Legionella pneumophila* | 37 | 9963 | *Streptococcus sp.* (group F) |
| 18 | 33270 | *Micromonas micros* | 38 | 8149 | *Haemophilus influenzae ,nontype* |
| 19 | 8193 | *Moraxella catarrhalis* | 39 | 9333 | *Haemophilus influenzae, nontype* |
| 20 | 6250 | *Neisseria meningitidis* | 40 | 49619 | *Streptococcus pneumoniae* |

| | | | | | |
|---|---|---|---|---|---|
| No.5 :4.3E5cfu/ml, No.34:7.0E5cfu/ml, Other: 1E6cfu/ml | | | | | |

### 2-3. Sandwich ELISA for detection of P4 antigen

Assay was carried out in almost the same manner as the aforementioned sandwich ELISA for detection of the P6 antigen. The anti-P4 PoAb (anti-P4 rec. PoAb No. 2) was immobilized onto an ELISA plate (5 µg/mL), followed by blocking with, for example, BSA. The immunogen or disrupted cells which had been appropriately diluted stepwise were added to the antibody-immobilized plate for reaction. After washing, the antigen-antibody complex was reacted with a biotin-labeled antibody prepared through labeling of the same type of PoAb as the immobilized anti-P4 PoAb. After washing again, HRP-labeled streptavidin was added to the plate for reaction, followed by color development with TMB. Figs. 4a and 4b show examples of dose-dependent curves. As shown therein, the P4 antigen was detected at 100 pg/mL or more, and disrupted NTHi cells were detected at 10³ to 10⁷ CFU/mL.

### 2-4. Cross-reactivity of sandwich ELISA for detection of P4 antigen

The cross-reactivity of the anti-P4 PoAb to a variety of bacteria shown in Table 1 was evaluated through the aforementioned sandwich ELISA. The results are shown in Fig. 5. All *Haemophilus influenzae* strains (including both encapsulated and non-encapsulated strains) were successfully detected.

Thus, a sandwich ELISA system capable of detecting mainly *Haemophilus influenzae* antigens was constructed by use of any of the above-produced anti-P6 PoAbs or anti-P4 PoAbs, which suggested that these PoAbs are possible to be used for construction of an immunochromatography assay system.

### 3. Construction of immunochromatography assay system for detection of P6 antigen, and evaluation of the performance thereof

### 3-1. Performance of sandwich immunochromatography employing anti-P6 PoAb

The gold-colloid-labeled anti-P6 PoAb prepared through the aforementioned procedure was diluted with, for example, a phosphate buffer containing a surfactant, and then mixed with the extracted P6 antigen. The resultant mixture was developed on an antibody-immobilized test strip through a technique as shown in Fig. 7. After washing with the aforementioned buffer, the signal intensity of each appeared test line was analyzed by means of a densitometer for quantification. The results are shown in Figs. 8a and 8b. As shown therein, in the case of the immunogen, the signal on the test line was detected at 1 ng/mL (minimum concentration in this assay) or more. In the case of disrupted NTHi cells, the signal on the test line was detected in a cell-concentration-dependent manner at 3 × 10⁴ CFU/mL or more. Thus, an immunochromatographic assay system capable of detecting *Haemophilus influenzae* antigens was constructed by use of the anti-P6 PoAb.

## Claims

1. An immunological assay method for detection of all *Haemophilus influenzae* strains, comprising employing an antibody which recognizes P4 antigen or P6 antigen of *Haemophilus influenzae*.

2. The method according to claim 1, wherein the antibody is a polyclonal antibody.

3. The method according to claim 1 or 2, which employs an ELISA system or an immunochromatography assay system.

4. An immunological assay device for detection of all *Haemophilus influenzae* strains, comprising an antibody which recognizes P4 antigen or P6 antigen of *Haemophilus influenzae*.

5. The assay device according to claim 4, wherein the antibody is a polyclonal antibody.

6. The assay device according to claim 4 or 5, which employs an ELISA system or an immunochromatography assay system.
